# FASCICULE DE BREVET EUROPEEN

(11) **EP 2 201 405 B1**
(45) Date de publication et mention de la délivrance du brevet: **07.11.2018**
(21) Numéro de dépôt: 08865635.0
(22) Date de dépôt: 08.10.2008
(51) Int. Cl.: G01T 1/161

(54) **DISPOSITIF DE DETECTION DE LA DESINTEGRATION DE RADIOISOTOPES DANS UN TISSU BIOLOGIQUE**
VORRICHTUNG ZUR ERKENNUNG DES ZERFALLS VON RADIOISOTOPEN IN BIOLOGISCHEM GEWEBE
DEVICE FOR DETECTING THE DISINTEGRATION OF RADIOISOTOPES IN BIOLOGICAL TISSUE

(30) Priorité: 12.10.2007 FR 0707176
(43) Date de publication de la demande: 30.06.2010
(73) Titulaire: Centre National de la Recherche Scientifique, 75794 Paris Cedex 16 (FR); Université d'Aix-Marseille, 13007 Marseille (FR); Université Paris-Sud 11, 91105 Orsay Cedex (FR)
(72) Inventeur: PINOT, Laurent, F-91510 Lardy (FR); GODART, Jérémy, F-78170 La Celle Saint Cloud (FR); DELPIERRE, Pierre-Auguste-Robert, F-13600 La Ciotat (FR); LANIECE, Philippe-Pierre-Louis, F-91190 Saint-Aubin (FR); DINKESPILER, Bernard, F-13830 La Bedoule (FR)
(74) Mandataire: IPAZ
(86) Numéro de dépôt international: PCT/FR2008/001407
(87) Numéro de publication internationale: WO 2009/080919

(56) Documents cités:
- JP-A- 2 206 786
- JP-A- 5 285 128
- US-A- 5 520 182
- US-A- 5 846 513
- US-A1- 2002 053 641
- US-A1- 2003 212 302
- US-A1- 2005 056 789
- PAIN F ET AL: "SIC: An Intracerebral Radiosensitive Probe for In Vivo Neuropharmacology Investigations in Small Laboratory Animals: Prototype Design, Characterization, and In Vivo Evaluation", IEEE TRANSACTIONS ON NUCLEAR SCIENCE, IEEE SERVICE CENTER, NEW YORK, NY, US, vol. 49, no. 3, 1 June 2002 (2002-06-01), XP011077641, ISSN: 0018-9499
- PAIN F ET AL: "SIC, an Intracerebral Radiosensitive Probe for In Vivo Neuropharmacology Investigations in Small Laboratory Animals: Theoretical Considerations and Physical Characteristics", IEEE TRANSACTIONS ON NUCLEAR SCIENCE, IEEE SERVICE CENTER, NEW YORK, NY, US, vol. 47, no. 1, 1 February 2000 (2000-02-01), XP011041651, ISSN: 0018-9499

## Description

La présente invention concerne un dispositif de détection de la désintégration de radioisotopes présents dans des tissus biologiques ou des organes, en particulier d'un animal d'expérimentation.

Le développement de plus en plus important de modèles animaux mimant des pathologies humaines telles que des maladies neurodégénératives ou bien tumorales, a ouvert de nouvelles opportunités dans les recherches menées dans des domaines aussi variés que la toxicologie, la pharmacologie, ou bien encore la biologie moléculaire. Cependant, le suivi *in vivo* de ces pathologies nécessite le développement de techniques d'imagerie adaptées aux contraintes spécifiques des études sur des petits animaux tels que, par exemple, des rongeurs.

Dans ce but, la plupart des modalités d'imagerie anatomiques et fonctionnelles utilisées couramment en clinique chez l'homme ont fait l'objet d'une adaptation au petit animal. Parmi ces techniques, la Tomographie par Emission de Positons (ci-après dénommée TEP) suscite un fort intérêt puisqu'elle est la seule à offrir une sensibilité proche du picomolaire, ce qui permet de réaliser des études sur les processus biochimiques ou moléculaires sans altérer les conditions normales ou pathologiques des tissus ou des organes étudiés.

Cette technique d'imagerie comprend l'injection d'un traceur radioactif par voie intraveineuse. Ce traceur possédant une activité biologique se fixe au niveau des tissus d'intérêt. La désintégration de l'atome radioactif présent dans le traceur conduit à l'émission d'un rayonnement β+, lequel après annihilation avec un électron du milieu, produit deux rayons γ en opposition qui sont détectés en coïncidence, par des capteurs situés à l'extérieur de l'animal.

A l'heure actuelle, les appareils connus montrent des limites propres à la technologie utilisée. En effet, ils requièrent une anesthésie de l'animal qui doit être immobilisé pour être placé entre les détecteurs. L'emploi d'un anesthésiant altère les paramètres physiologiques de l'organisme de l'animal et donc complique l'analyse de l'activité biologique du traceur. De plus, en vue de l'utilisation chez l'homme de molécules testées chez l'animal, il est préférable de se rapprocher des conditions cliniques, dans lesquelles on ne pratique en général aucune anesthésie. Ces tomographes présentent également une faible sensibilité due à la nécessité de détecter deux rayonnements en coïncidence et aux faibles angles solides dus à la géométrie en anneau des capteurs. Cette faible sensibilité leur confère une faible résolution temporelle, peu adaptée à la détermination de paramètres cinétiques desquels découlent de nombreux paramètres physiologiques. Enfin ces détecteurs sont extrêmement coûteux.

Afin de remédier aux inconvénients précités, deux nouvelles approches sont actuellement en cours de développement.

La première consiste en une mini-camera TEP spécifiquement développée pour l'imagerie du cerveau du rat et destinée à être fixée sur la tête de l'animal. Ce dispositif repose sur l'utilisation de photodiodes à avalanche, une électronique spécifique étant associée à chaque élément de détection. Il entoure la tête de l'animal et comprend un système de contre-poids qui compense la masse du détecteur. Ce dispositif est relativement encombrant et lourd du fait de la masse du détecteur d'environ 150 g et de la nécessité d'avoir un lien physique entre le détecteur et une unité d'analyse et de stockage, ce qui ne permet pas d'assurer une complète liberté à l'animal durant l'expérimentation et limite les études comportementales réalisables. Enfin, le parcours des particules β+ dans les tissus étant limité, l'information provient de la détection en coïncidence des deux rayonnements γ issus de l'annihilation d'une particule β+ avec son anti-particule l'électron, par un anneau de capteurs situé à l'extérieur de l'animal, ce qui confère à ce dispositif les mêmes limites en sensibilité que les TEP conventionnels.

La seconde approche repose sur l'utilisation d'une sonde miniaturisée, implantable directement dans le tissu cérébral du rongeur (voir le document PAIN F. ET AL: "SIC: An Intracerebral Radiosensitive Probe for In Vivo Neuropharmacology Investigations in Small Laboratory Animais: Prototype Design, Characterization, and In Vivo Evaluation", IEEE TRANSACTIONS ON NUCLEAR SCIENCE, vol. 49, no. 3). Elle comprend une fibre optique plastique scintillante reliée via un guide optique à un photodétecteur bas bruit. Cette sonde permet de s'affranchir des problèmes d'anesthésie ou de contention. Elle possède une haute sensibilité puisqu'elle est directement placée au contact de la zone de mesure. A la différence des appareils à détecteurs externes reposant sur la mesure en coïncidence de rayonnements γ d'annihilation des positons avec les électrons, la sonde mesure directement les positons (ou rayonnement β+) émis, ce qui améliore la sensibilité du détecteur et donc son aptitude à établir des cinétiques précises des radiotraceurs. Elle peut de même être appliquée à la détection de rayonnement β- ou α, ce qui étend le champ de radiotraceurs utilisables. Enfin, ce type de dispositif s'avère peu coûteux et simple d'utilisation en comparaison des dispositifs de détection externes.

Si ce système s'avère très intéressant, il n'en possède pas moins des limitations inhérentes à la technologie utilisée, l'utilisation d'une fibre optique scintillante comme capteur radiosensible limitant la mesure à un simple comptage du nombre des rayonnements. De plus, l'animal n'est pas libre de ses mouvements pendant la mesure, car les photodétecteurs sont trop encombrants pour être placés sur l'animal, ce qui impose de déporter le photodétecteur et de le relier à la sonde par un guide optique. Enfin, la sensibilité du capteur à la lumière impose de l'utiliser dans l'obscurité, ce qui complique fortement les conditions d'expérimentation.

L'invention a notamment pour but d'apporter une solution simple, économique et efficace à ces différents problèmes.

Elle propose à cet effet, un dispositif implantable dans le cerveau en particulier d'un animal pour la détection d'un rayonnement ionisant émis par désintégration spontanée d'un radioisotope, caractérisé en ce qu'il comprend un détecteur implantable en forme d'aiguille dont une extrémité destinée à être implantée porte au moins une rangée de détecteurs élémentaires orientée dans la direction d'implantation de l'aiguille, l'autre extrémité de l'aiguille étant solidaire d'un circuit imprimé comprenant un ensemble de circuits d'amplification, de mise en forme, de comptage et de transmission à distance sans fil, qui sont reliés aux détecteurs élémentaires et qui sont destinés à être portés en particulier par un animal, celui-ci étant éveillé et libre de ses déplacements, l'aiguille étant formée d'un substrat en matériau semi-conducteur portant une pluralité d'électrodes formant les détecteurs élémentaires, en ce que l'ensemble de l'aiguille, du circuit imprimé et du circuit intégré de traitement du signal a une masse inférieure à 1g, le circuit imprimé ayant une surface inférieure à 1 cm², et l'aiguille ayant une longueur de l'ordre de 1 à 2 cm, une épaisseur comprise entre 200 et 500 µm, et une masse inférieure à 100 mg.

L'utilisation d'un détecteur en matériau semi-conducteur implanté à l'intérieur du corps de l'animal permet une conversion directe de l'énergie du rayonnement issu de la désintégration en un courant électrique mesurable *in situ*. Le signal est ensuite transmis à un circuit d'amplification. Un circuit de mise en forme permet d'éliminer une partie des signaux parasites résultant du bruit électronique et du bruit de photons parasites. Le signal est ensuite transmis à distance par liaison sans fil pour analyse et traitement.

L'aiguille peut être formée d'un substrat en matériau semi-conducteur à haute résistivité.

Selon une autre caractéristique de l'invention, chaque détecteur élémentaire est relié à sa propre chaîne de traitement du signal qui fait partie d'un circuit intégré porté par le circuit imprimé et comportant des moyens d'amplification, de conversion, de filtrage, de seuillage et de comptage des signaux des détecteurs élémentaires.

La liaison de chaque détecteur élémentaire à sa propre chaîne électronique permet la collecte des signaux en provenance des détecteurs élémentaires en fonction du temps, ce qui permet d'envisager la réalisation a posteriori de cartes de la biodistribution de la fixation d'un traceur radioactif au sein de la zone analysée et de suivre l'évolution de cette distribution pour réaliser des mesures cinétiques précises du fait de l'amélioration de la sensibilité du détecteur.

L'ensemble du dispositif comprenant le détecteur implantable dans le cerveau de l'animal ainsi que les différents circuits de traitement est destiné à être porté par l'animal qui reste totalement libre de ses mouvements, ce qui limite considérablement le niveau de stress de l'animal et facilite les expérimentations.

Le matériau utilisé pour le substrat et les détecteurs peut être du silicium de haute résistivité, par exemple. Dans ce cas, les détecteurs élémentaires sont des diodes polarisées en inverse. L'utilisation d'un matériau à haute résistivité permet d'éviter les circulations de courants de fuite ou parasites au sein des diodes.

Les détecteurs élémentaires sont polarisés à une tension électrique permettant une déplétion totale de chaque détecteur élémentaire.

Avantageusement, l'aiguille a une section sensiblement rectangulaire et porte sur une de ses faces les détecteurs élémentaires.

La face extérieure de chaque détecteur élémentaire et la face du substrat opposée aux détecteurs élémentaires sont recouvertes chacune d'une électrode métallique.

Les détecteurs élémentaires ont une largeur et une longueur comprises entre 100 µm et 1 mm, par exemple une largeur de 200 µm et une longueur de 500 µm.

Les détecteurs élémentaires sont reliés aux moyens d'amplification par des pistes de connexion, lesquelles peuvent être séparées par des lignes conductrices reliées à la masse, ce qui permet de limiter les phénomènes de diaphonie et de couplage capacitif entre les pistes.

Selon une autre caractéristique de l'invention, un anneau conducteur entoure l'ensemble des détecteurs élémentaires et permet de stabiliser les lignes du champ électrique dans la zone de déplétion.

Le circuit imprimé est relié par un ensemble de conducteurs, qui peuvent être sous-cutanés, à un module d'alimentation électrique, de commande et de transmission à distance, destiné à être fixé sur le dos de l'animal et ayant des dimensions de l'ordre du centimètre.

L'alimentation électrique peut être réalisée au moyen de piles électriques, par radiofréquence, par des cellules photovoltaïques ou par des photodiodes.

Le module de transmission à distance peut comprendre un système radiofréquence bidirectionnel, ou un système optique tel que par exemple un système optique infrarouge.

Selon une autre caractéristique de l'invention, le détecteur est recouvert par une couche de protection imperméable, opaque, biocompatible et électriquement isolante. Une telle couche permet de protéger le détecteur de l'humidité des tissus environnants et assure une protection contre les photons parasitant les détecteurs. L'isolation électrique permet de garantir un fonctionnement optimal de chacun des détecteurs élémentaires et une transmission sans perturbation du signal par les pistes de connexion au circuit de traitement. La biocompatibilité de la couche de protection permet de limiter d'éventuelles réactions inflammatoires qui peuvent altérer les paramètres physiologiques du tissu étudié et introduire un biais dans les expérimentations.

Avantageusement, cette couche comprend une première couche opaque et électriquement isolante et une deuxième couche recouvrant la première et qui est biocompatible et étanche. La première couche est un revêtement du type vernis et a une épaisseur de l'ordre de quelques micromètres et la deuxième couche est un polymère plastique du type polystyrène et a une épaisseur de l'ordre de 5 à 10 µm.

Le dispositif selon l'invention est destiné en particulier à la détection des rayonnements β⁺, β⁻ ou α pour l'analyse de la distribution et de la fixation d'un radiotraceur dans les tissus avec une résolution temporelle de l'ordre de la seconde. En effet, les détecteurs utilisés dans le dispositif permettent d'utiliser des radiotraceurs émetteurs α ou β⁻, sans se limiter aux isotopes émetteurs de β⁺, ce qui présente de l'intérêt pour le développement de nouvelles familles de radiotraceurs.

Le dispositif peut être implanté dans le cerveau de tout type d'animal et en particulier dans celui d'un petit animal tel qu'un rat ou dans un cerveau humain.

Les détecteurs élémentaires agencés à une extrémité de l'aiguille peuvent être du type CMOS ou 3D.

D'autres avantages et caractéristiques de l'invention apparaîtront à la lecture de la description suivante faite à titre d'exemple non limitatif et en référence aux dessins annexés dans lesquels :
- la figure 1 est une vue schématique en coupe du dispositif selon l'invention, comprenant un détecteur implanté dans le crâne d'un animal d'expérimentation ;
- la figure 2 est une vue schématique en perspective d'un animal d'expérimentation portant le dispositif de la figure 1 relié à une unité d'analyse ;
- la figure 3 est une vue schématique en coupe axiale du détecteur implanté dans un tissu ;
- la figure 4 est une vue schématique de dessus du détecteur de la figure 3.

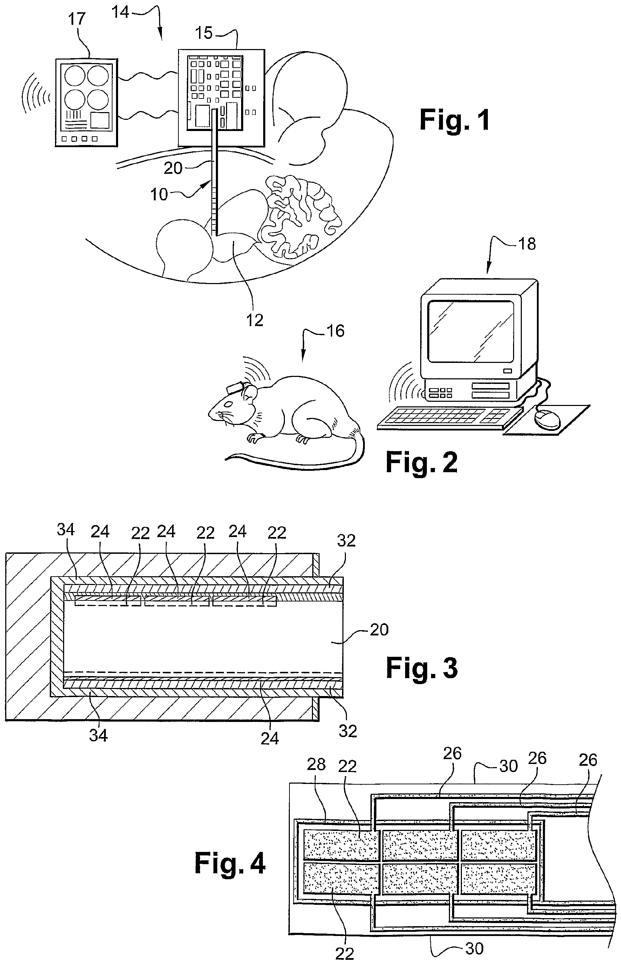

Comme représenté en figures 1 et 2, le dispositif selon l'invention comprend des moyens 10 de détection d'un rayonnement β ou α, en matériau semi-conducteur, implantés dans le crâne 12 d'un animal d'expérimentation, et des moyens 14 de traitement du signal dont certains sont montés sur un circuit imprimé 15 solidaire des moyens de détection 10 et relié à un module 17 d'alimentation et de transmission à distance vers une station d'analyse 18, le module 17 étant fixé sur l'animal à faible distance du circuit imprimé 15.

La description qui suit est faite en référence aux figures 3 et 4. Les moyens de détection 10 comprennent une aiguille 20 en matériau semi-conducteur dont l'extrémité implantée comporte un ensemble de détecteurs élémentaires 22 également en matériau semi-conducteur (seuls trois détecteurs élémentaires sont visibles en figure 3). Le substrat 20 formant l'aiguille peut être en silicium de haute résistivité dopé n tandis que les détecteurs élémentaires 22 sont dopés p afin de former une pluralité de diodes de détection polarisées en inverse. La tension de polarisation appliquée est telle qu'elle assure une déplétion totale de chacun des détecteurs élémentaires 22 dans le but d'avoir un volume de détection maximum du rayonnement traversant les détecteurs élémentaires 22.

Le substrat 20 a une section de forme rectangulaire et les détecteurs élémentaires 22 sont portés par une face du substrat. Les détecteurs élémentaires 22 sont alignés dans le sens de pénétration du détecteur 10 dans le crâne 12 de l'animal 16.

La face extérieure de chaque détecteur élémentaire 22 et la face du substrat 20 opposée aux détecteurs élémentaires 22 sont recouvertes chacune d'une électrode métallique 24, par exemple, en aluminium et d'une épaisseur d'environ 1 µm.

La figure 4 est une vue de dessus des détecteurs élémentaires 22 et une partie du substrat 20 sur lequel ils sont fixés. Deux rangées parallèles de trois détecteurs élémentaires 22 chacune sont agencées à l'extrémité implantée de l'aiguille. Les détecteurs élémentaires 22 ont une forme rectangulaire et sont alignés dans le sens de leur longueur le long de l'aiguille afin que le substrat 20 présente une section réduite pour rendre l'opération chirurgicale d'implantation de l'aiguille dans le tissu la moins traumatisante possible pour l'animal 16.

Les détecteurs élémentaires 22 sont reliés par des pistes 26 aux moyens 14 de traitement portés par le circuit imprimé 15 et sont entourés au plus près par un anneau conducteur 28 qui permet de stabiliser les lignes du champ électrique à l'intérieur de la zone active de détection de chacun des détecteurs élémentaires 22. La zone de découpe 30 du substrat est positionnée à une distance suffisante de l'anneau 28 afin de minimiser les phénomènes de courant de fuite dus à la diminution de la résistivité au niveau de la zone de découpe 30 du fait des modifications de la structure cristalline du substrat en ces endroits. Typiquement, la distance entre la zone de découpe 30 et l'anneau 28 correspond à l'épaisseur du substrat, c'est-à-dire à la dimension de la section du substrat 20 dans la direction perpendiculaire aux détecteurs élémentaires 22. Cependant, un compromis peut être réalisé entre compacité et courant de fuite afin d'avoir une distance entre l'anneau 28 et la découpe inférieure à l'épaisseur du substrat 20.

L'espace entre l'anneau conducteur 28 et la zone de découpe 30 est utilisé pour le passage des différentes pistes 26 de connexion aux détecteurs élémentaires 22, ce qui garantit une compacité maximale au détecteur.

Des lignes de masse, non représentées, peuvent être réalisées entre chacune des pistes 26 afin de limiter les phénomènes de couplage capacitif ou de diaphonie entre les pistes 26 lesquelles sont réalisées en matériau métallique et, de manière similaire aux électrodes, en aluminium.

L'invention permet d'assurer un fonctionnement du dispositif dans des conditions standard de laboratoire, et notamment sous un éclairage normal. Pour cela, le substrat 20 est recouvert d'une couche opaque 32, typiquement à base de vernis, qui assure une protection contre la lumière visible parvenant jusqu'aux diodes. Une telle couche 32 assure également une isolation électrique des détecteurs élémentaires 22 et de leurs pistes 26.

Le détecteur est ensuite recouvert d'une deuxième couche 34 biocompatible avec l'environnement dans lequel le détecteur est implanté. La protection choisie est obtenue par évaporation d'un polymère en four puis repolymérisation sur le détecteur recouvert de la première couche de protection, dans une chambre à température ambiante. Ce procédé permet d'assurer un dépôt homogène de la couche biocompatible 34 sur le détecteur. Le dépôt d'un polymère assure également une protection du détecteur contre l'humidité du milieu d'implantation, qui peut induire un bruit supplémentaire au niveau des pistes 26 du dispositif. L'épaisseur de cette deuxième couche ne doit pas être trop importante afin de ne pas absorber les rayonnements de désintégration. Le polymère est par exemple du polystyrène et son épaisseur est de l'ordre de 5 à 10 µm.

L'ensemble formé par le circuit imprimé 15, l'aiguille de détection 20 et les moyens 14 de traitement du signal portés par le circuit 15 a une masse inférieure à 1 g. La surface du circuit imprimé 15 a une surface inférieure à 1 cm².

Le détecteur ainsi formé est préalablement implanté dans la zone d'étude par stéréotaxie. L'ensemble est par la suite solidarisé sur le crâne de l'animal, par exemple, par un système mécanique tel un casque ou une lanière ou bien par un ciment. Cependant, l'opérateur peut choisir de ne pas solidariser le détecteur dans le cas d'applications spécifiques de courte durée, en le laissant fixé sur le système de stéréotaxie.

Chaque détecteur élémentaire 22 est relié à sa propre chaîne de traitement de signal, qui comprend des circuits d'amplification, de conversion, de filtrage, de seuillage et de comptage miniaturisés et faisant partie d'un circuit intégré porté par le circuit imprimé 15 qui est solidaire de l'extrémité externe de l'aiguille de détection et qui est relié au module 17 d'alimentation électrique, de commande et de transmission sans fil. Le circuit intégré réalisé dans une technologie submicronique est résistant aux radiations ionisantes.

Les signaux transmis par les pistes 26 au circuit imprimé 15 sont amplifiés grâce à des amplificateurs de charge puis convertis en tension. Les circuits de seuillage permettent de ne laisser passer vers les circuits de comptage que les signaux correspondant à des rayonnements d'énergie supérieure à un seuil réglable par un opérateur. Cela permet d'éliminer le bruit électronique et d'optimiser le nombre de particules β comptées par rapport au bruit du aux photons parasites.

En sortie du circuit imprimé, les signaux numériques sont transmis à distance par le module 17. La transmission peut être réalisée en utilisant un système radiofréquence bidirectionnel ou un système optique tel que par exemple un système optique infrarouge.

L'alimentation électrique du dispositif peut être réalisée au moyen de deux piles électriques de 1,5 Volts permettant une utilisation du détecteur sur une longue période, par exemple de l'ordre d'une centaine d'heures. D'autres types d'alimentation peuvent être utilisés tels que des systèmes à radiofréquence, à cellules photovoltaïques ou à photodiodes. Un système élévateur de tension peut être utilisé pour obtenir à partir de la tension délivrée par les piles, la tension de polarisation nécessaire aux détecteurs, de l'ordre de quelques dizaines de volts. Ce système élévateur de tension peut être réalisé par une pompe de charges. Le module 17 d'alimentation électrique, de commande et de transmission à distance peut être fixé en arrière du lieu d'implantation du détecteur, par exemple sur le cou ou le dos de l'animal 16 par un petit sac à dos ou bien par des lanières. Ce module a des dimensions de l'ordre de 1 cm², ce qui garantit une totale liberté de l'animal durant les expérimentations.

Le circuit imprimé 15 peut être relié au module 17 par un mini-câble lequel peut être externe ou sous-cutané pour éviter tout arrachement par l'animal en cours d'expérimentation.

Le dispositif fonctionne de la manière suivante : un traceur radioactif injecté par voie intraveineuse dans le corps de l'animal se fixe dans un tissu d'intérêt à proximité du détecteur. Le rayonnement émis par désintégration spontanée du radioisotope traverse le détecteur et induit un dépôt de charges par ionisation qui est proportionnel à l'énergie déposée par le rayonnement dans la zone déplétée. Le signal de charge est transmis aux circuits de traitement pour amplification, conversion, filtrage, seuillage, comptage, puis est transmis à une unité 18 d'analyse et de post-traitement située par exemple à plusieurs mètres de l'animal 16.

De nombreuses variantes du dispositif décrit peuvent être envisagées. Par exemple, on peut utiliser un nombre plus important de détecteurs élémentaires 22 que celui représenté aux dessins. L'aiguille peut ainsi comporter, par exemple, deux rangées de 10 détecteurs chacune.

Le substrat 20 présente typiquement une épaisseur de l'ordre de 200 à 500 µm, une largeur de l'ordre de 1 mm, une longueur de l'ordre de 1 à 2 cm pour une masse inférieure à 100 mg. Les détecteurs élémentaires ont une largeur et une longueur comprise entre 100 µm et 1 mm. Par example, l'épaisseur du substrat est de l'ordre de 500 µm, la largeur et la longueur des détecteurs élémentaires étant de 200 µm et 500 µm, respectivement.

La distance inter-détecteurs est de l'ordre de 20 µm et la distance séparant deux pistes de connexion est de l'ordre de 10 µm.

La tension de polarisation des détecteurs élémentaires 22 est de l'ordre de quelques dizaines de volts.

Le matériau semi-conducteur peut être en silicium à haute résistivité.

Le dispositif selon l'invention permet la détection de rayonnement β+ et de rayonnement α ou β-, selon le radioisotope utilisé. Il permet une mesure précise de l'évolution temporelle de l'activité du rayonnement dans une zone tissulaire d'intérêt, pour un sujet éveillé et totalement libre de ses mouvements du fait de l'extrême compacité du détecteur couplé à une électronique miniature totalement autonome. De plus, la détection directe du rayonnement β, au lieu du rayonnement γ issu du processus d'annihilation, permet d'améliorer la sensibilité et d'obtenir une résolution temporelle de l'ordre de la seconde, ce qui améliore la précision des mesures cinétiques. Enfin, la possibilité de détecter non seulement les rayonnements β⁺, mais également les rayonnements β- et α permet d'étendre le champ d'application du dispositif à de nouveaux radiotraceurs émetteurs α ou β⁻.

Le dispositif selon l'invention peut être utilisé en combinaison avec un second dispositif identique, l'un des dispositifs étant implanté dans une zone d'étude, l'autre étant implanté dans une zone témoin. L'analyse de l'évolution cinétique de la différence du signal provenant de la zone témoin avec le signal provenant de la zone d'étude permet de renseigner sur le niveau d'activité biologique spécifique à la zone d'étude.

Le dispositif selon l'invention n'est pas limité à l'exploration fonctionnelle des tissus de la boite crânienne. Il est en effet tout à fait envisageable d'implanter un détecteur dans les tissus d'autres organes de l'animal où l'on désire évaluer la fixation d'un radiotraceur. De même, le dispositif selon l'invention peut être utilisé sur des animaux de taille plus importante que les rongeurs, ou sur l'homme.

Le dispositif selon l'invention peut également être utilisé en combinaison avec des détecteurs élémentaires de type CMOS (Complementary Metal Oxide Semi-Conductor) dont les dimensions peuvent être réduites jusqu'à des valeurs de l'ordre de 10 µm. Dans ce cas, la tension de polarisation appliquée aux détecteurs élémentaires peut être plus faible qu'avec des diodes et le dispositif ne requiert pas d'anneau conducteur 28.

Le dispositif selon l'invention peut également être utilisé en combinaison avec des détecteurs semi-conducteurs de type "3D" où les électrodes qui établissent le champ électrique de déplétion sont des trous métallisés pratiqués dans le substrat. Dans ce cas, le dispositif ne requiert pas d'anneau conducteur 28.

## Revendications

1. Dispositif implantable dans le cerveau en particulier d'un animal (16) pour la détection d'un rayonnement ionisant émis par désintégration spontanée d'un radioisotope, le dispositif comprenant un détecteur implantable (10) en forme d'aiguille dont une extrémité destinée à être implantée porte au moins une rangée de détecteurs élémentaires (22) orientée dans la direction d'implantation de l'aiguille, l'autre extrémité de l'aiguille étant solidaire d'un circuit imprimé (15), le dispositif comprenant un ensemble (14) de circuits d'amplification, de mise en forme, de comptage et de transmission à distance sans fil, qui sont reliés aux détecteurs élémentaires (22) et qui sont destinés à être portés en particulier par un animal (16), celui-ci étant éveillé et libre de ses déplacements, **caractérisé en ce que** l'aiguille est formée d'un substrat (20) en matériau semi-conducteur portant une pluralité d'électrodes formant les détecteurs élémentaires (22), **en ce que** l'ensemble de l'aiguille (20), du circuit imprimé (15) et du circuit intégré de traitement du signal (14) a une masse inférieure à 1g, le circuit imprimé (15) ayant une surface inférieure à 1 cm², et **en ce que** l'aiguille a une longueur de l'ordre de 1 à 2 cm, une épaisseur comprise entre 200 et 500 µm, et une masse inférieure à 100 mg.

2. Dispositif selon la revendication 1, **caractérisé en ce que** l'aiguille est formée d'un substrat (20) en matériau semi-conducteur à haute résistivité.

3. Dispositif selon la revendication 1 ou 2, **caractérisé en ce que** chaque détecteur élémentaire (22) est relié à sa propre chaîne de traitement du signal qui fait partie d'un circuit intégré porté par le circuit imprimé (15) et comportant des moyens d'amplification, de conversion, de filtrage, de seuillage et de comptage des signaux des détecteurs élémentaires (22).

4. Dispositif selon la revendication 3, **caractérisé en ce que** les détecteurs élémentaires (22) sont polarisés à une tension électrique permettant une déplétion totale de chaque détecteur élémentaire (22).

5. Dispositif selon la revendication 3 ou 4, **caractérisé en ce que** l'aiguille a une section sensiblement rectangulaire et porte sur une de ses faces les détecteurs élémentaires (22).

6. Dispositif selon la revendication 5, **caractérisé en ce que** la face extérieure de chaque détecteur élémentaire (22) et la face du substrat (20) opposée aux détecteurs élémentaires (22) sont recouvertes chacune d'une électrode métallique (24).

7. Dispositif selon l'une des revendications 3 à 6, **caractérisé en ce que** des pistes de connexion (26) relient les détecteurs élémentaires (22) aux moyens d'amplification et sont séparées par des lignes conductrices reliées à la masse.

8. Dispositif selon l'une des revendications 3 à 7, **caractérisé en ce qu'**un anneau conducteur (28) entoure l'ensemble des détecteurs élémentaires (22).

9. Dispositif selon l'une des revendications 3 à 8, **caractérisé en ce que** le circuit imprimé (15) solidaire de l'aiguille est relié par un ensemble de conducteurs à un module (17) d'alimentation électrique, de commande et de transmission à distance sans fil, destiné à être fixé sur le dos en particulier d'un animal (16) ou une autre partie de son corps et ayant des dimensions de l'ordre du centimètre.

10. Dispositif selon l'une des revendications 3 à 9, **caractérisé en ce que** le circuit imprimé (15) solidaire de l'aiguille est destiné à être relié au module d'alimentation, de commande et de transmission par une liaison filaire sous-cutanée.

11. Dispositif selon la revendication 9 ou 10, **caractérisé en ce que** le module de transmission à distance comprend un système radiofréquence bidirectionnel, ou un système optique tel que par exemple un système optique infrarouge.

12. Dispositif selon l'une des revendications 2 à 11, **caractérisé en ce que** le substrat (20) et les détecteurs élémentaires (22) sont réalisés en silicium à haute résistivité.

13. Dispositif selon l'une des revendications précédentes, **caractérisé en ce que** le détecteur (10) est recouvert par une couche de protection imperméable, opaque, biocompatible et électriquement isolante.

14. Dispositif selon la revendication 13, **caractérisé en ce que** la couche de protection comprend une première couche (32) opaque et électriquement isolante telle qu'un revêtement du type verni et une deuxième couche (34) qui recouvre la première et est biocompatible et étanche, telle par exemple qu'un polymère plastique du type polystyrène.

15. Dispositif selon l'une des revendications précédentes, **caractérisé en ce qu'**il est destiné à être implanté dans le cerveau d'un petit animal tel qu'un rat par exemple ou dans le cerveau d'un grand animal, ou dans un cerveau humain.

## Patentansprüche

1. Implantierbare Vorrichtung in das Gehirn insbesondere eines Tieres (16) zur Erkennung einer ionisierenden, durch spontanen Zerfall eines Radioisotops emittierten Strahlung, umfassend einen implantierbaren Detektor (10) in Form einer Nadel, deren ein zur Implantation eingerichtetes Ende mindestens eine in Implantationsrichtung der Nadel ausgerichtete Reihe von elementaren Detektoren (22) trägt, wobei das andere Ende der Nadel mit einer Leiterplatte (15) verbunden ist, wobei die Vorrichtung eine Anordnung (14) von Schaltungen zur Verstärkung, Formgebung, Zählung und drahtlosen Fernübertragung umfasst, welche mit den elementaren Detektoren (22) verbunden und dazu eingerichtet sind, insbesondere von einem Tier (16) getragen zu werden, wobei dieses im Wachzustand ist und dessen Bewegungen uneingeschränkt sind, **dadurch gekennzeichnet, dass** die Nadel aus einem Substrat (20) aus einem Halbleitermaterial mit einer Vielzahl von Elektroden besteht, welche die elementaren Detektoren (22) ausbilden, dass die Anordnung aus der Nadel (20), der Leiterplatte (15) und der integrierten Signalverarbeitungsschaltung (14) eine Masse kleiner als 1g hat, wobei die Leiterplatte (15) eine Oberfläche kleiner als 1 cm² aufweist, und dass die Nadel eine Länge in Höhe von 1 bis 2 cm, eine Dicke zwischen 200 und 500 µm und eine Masse kleiner als 100 mg aufweist.

2. Vorrichtung nach Anspruch 1, **dadurch gekennzeichnet, dass** die Nadel aus einem Substrat (20) aus einem hochwiderstandsfähigen Halbleiter besteht.

3. Vorrichtung nach Anspruch 1 oder 2, **dadurch gekennzeichnet, dass** jeder elementare Detektor (22) mit seiner eigenen Signalverarbeitungskette verbunden ist, welche zu einer integrierten Schaltung gehört, die an der Leiterplatte (15) angebracht ist und Mittel zur Verstärkung, zur Umwandlung, zur Filtrierung, zum Schwellenwertvergleich und zur Zählung der Signale der elementaren Detektoren (22) umfasst.

4. Vorrichtung nach Anspruch 3, **dadurch gekennzeichnet, dass** die elementaren Detektoren (22) zu einer elektrischen Spannung polarisiert sind, welche eine vollständige Verarmung jedes elementaren Detektors (22) ermöglicht.

5. Vorrichtung nach Anspruch 3 oder 4, **dadurch gekennzeichnet, dass** die Nadel einen im Wesentlichen rechteckigen Querschnitt aufweist und die elementaren Detektoren (22) auf einer seiner Seiten angebracht sind.

6. Vorrichtung nach Anspruch 5, **dadurch gekennzeichnet, dass** die Außenseite jedes elementaren Detektors (22) und die den elementaren Detektoren (22) gegenüberliegenden Seite des Substrats (20) jeweils mit einer Metallelektrode (24) überzogen sind.

7. Vorrichtung nach einem der Ansprüche 3 bis 6, **dadurch gekennzeichnet, dass** Anschlussbahnen (26) die elementaren Detektoren (22) mit den Verstärkungsmitteln verbinden und durch an der Masse angeschlossene Leiterleitungen getrennt angeordnet sind.

8. Vorrichtung nach einem der Ansprüche 3 bis 7, **dadurch gekennzeichnet, dass** ein Leiterring (28) die Anordnung der elementaren Detektoren (22) umgibt.

9. Vorrichtung nach einem der Ansprüche 3 bis 8, **dadurch gekennzeichnet, dass** die mit der Nadel verbundene Leiterplatte (15) durch eine Anordnung von Leitern mit einem Modul (17) zur elektrischen Versorgung, zur Steuerung und zur drahtlosen Fernübertragung angeschlossen ist, welches zur Befestigung an dem Rücken insbesondere eines Tieres (16) oder an einem anderen Teil seines Körpers eingerichtet ist und Abmessungen in cm Größe aufweist.

10. Vorrichtung nach einem der Ansprüche 3 bis 9, **dadurch gekennzeichnet, dass** die mit der Nadel verbundene Leiterplatte (15) dazu eingerichtet ist, über eine subkutane Drahtverbindung an das Modul zur Stromversorgung, Steuerung und Übertragung angeschlossen zu werden.

11. Vorrichtung nach Anspruch 9 oder 10, **dadurch gekennzeichnet, dass** das Modul zur Fernübertragung ein bidirektionales Radiofrequenzsystem oder ein optisches System wie zum Beispiel ein optisches Infrarotsystem umfasst.

12. Vorrichtung nach einem der Ansprüche 2 bis 11, **dadurch gekennzeichnet, dass** das Substrat (20) und die elementaren Detektoren (22) aus hochwiderstandsfähigem Silicium ausgeführt sind.

13. Vorrichtung nach einem der vorhergehenden Ansprüche, **dadurch gekennzeichnet, dass** der Detektor (10) mit einer undurchlässigen, trüben, biokompatiblen und elektrisch isolierenden Schutzschicht überzogen ist.

14. Vorrichtung nach Anspruch 13, **dadurch gekennzeichnet, dass** die Schutzschicht eine erste trübe und elektrisch isolierende Schicht (32) wie eine lackartige Beschichtung und eine zweite, die erste Schicht überdeckende, biokompatible und undurchlässige Schicht (34) wie zum Beispiel ein polystyrenartiges Kunststoffpolymer umfasst.

15. Vorrichtung nach einem der vorhergehenden Ansprüche, **dadurch gekennzeichnet, dass** sie dazu eingerichtet ist, in das Gehirn eines kleinen Tieres wie zum Beispiel einer Ratte oder in das Gehirn eines großen Tieres oder in ein menschliches Gehirn implantiert zu werden.

## Claims

1. Device which is implantable into the brain, in particular of an animal (16), for detecting ionizing radiation emitted via spontaneous disintegration of a radioisotope, wherein it includes a needle-shaped implantable detector (10) one end of which is intended to be implanted and bears at least one row of basic detectors (22) oriented in the direction of implantation of the needle, the other end of the needle being secured to a printed circuit (15), said device comprising a set (14) of amplification, shaping, counting and wireless remote transmission circuits, which are connected to the basic detectors (22) and which are intended to be worn in particular by the animal (16), the latter being awake and free to move about, **characterized in that** the needle is formed from a substrate (20) made of semiconductor material and bearing a plurality of electrodes forming the basic detectors (22), wherein the assembly of the needle (20), the printed circuit (15) and the signal-processing integrated circuit (14) has a weight of less than 1 g, the printed circuit (15) having a surface area of less than 1 cm², wherein the needle has a length of the order of 1 to 2 cm, a thickness of between 200 and 500 µm and a weight of less than 100 mg.

2. Device of claim 1, wherein the needle is formed from a substrate (20) made of a high-resistivity semiconductor material.

3. Device as claimed in claims 1 or 2, wherein each basic detector (22) is connected to its own signal processing chain which forms part of an integrated circuit included in the printed circuit (15) and comprising means for amplifying, converting, filtering, thresholding and counting the signals from the basic detectors (22).

4. Device of claim 3, wherein the basic detectors (22) are biased at an electric voltage enabling complete depletion of each basic detector (22).

5. Device as claimed in claims 3 or 4, wherein the needle has a substantially rectangular cross-section and, on one of the faces thereof, holds the basic detectors (22).

6. Device of claim 5, wherein the exterior face of each basic detector (22) and the face of the substrate (20) opposite the basic detectors (22) are each covered by a metallic electrode (24).

7. Device as claimed in claims 3 to 6, wherein connecting tracks (26) connect the basic detectors (22) to the amplification means and are separated by grounded conducting lines.

8. Device as claimed in claims 3 to 7, wherein a conducting ring (28) surrounds all of the basic detectors (22).

9. Device as claimed in claims 3 to 8, wherein the printed circuit (15) secured to the needle is connected via a set of conductors to an electric power supply, control and remote wireless transmission module (17) intended to be fastened onto the back of the animal (16) or another portion of the body thereof and having dimensions of the order of a centimetre.

10. Device as claimed in claims 3 to 9, wherein the printed circuit (15) secured to the needle is intended to be connected to the power supply, control and transmission module via a subcutaneous wire connection.

11. Device as claimed in claims 9 or 10, wherein the remote transmission module includes a bidirectional radiofrequency system or an optical system, e.g., such as an infrared optical system.

12. Device as claimed in claims 2 to 11, wherein the substrate (20) and the basic detectors (22) are made of high-resistivity silicon.

13. Device as claimed in any of the preceding claims, wherein the detector (10) covered by an impermeable, opaque, biocompatible and electrically insulating protective layer.

14. Device of claim 13, wherein the protective layer includes a first opaque and electrically insulating layer (32) such as a varnish-type coating, and a second layer (34) which covers the first and which is biocompatible and watertight, e.g., such as a plastic polymer of the polystyrene type.

15. Device as claimed in any of the preceding claims, wherein it is intended to be implanted into the brain of a small animal such as a rat, for example, or into the brain of a large animal, or into a human brain.
